# NEUE EUROPÄISCHE PATENTSCHRIFT

(11) **EP 2 604 295 B2**
(45) Veröffentlichungstag und Bekanntmachung des Hinweises auf die Entscheidung über den Einspruch: **07.09.2022**
(45) Hinweis auf die Patenterteilung: 27.05.2015
(21) Anmeldenummer: 12197251.7
(22) Anmeldetag: 14.12.2012
(51) Int. Cl.: A61L 2/20, B65B 55/18, B67C 7/00

(54) **Verfahren zum Sterilisieren von Behältnissen**
Method for sterilising containers
Procédé de stérilisation de récipients

(30) Priorität: 14.12.2011 DE 102011056440
(43) Veröffentlichungstag der Anmeldung: 19.06.2013
(73) Patentinhaber: Krones AG, 93073 Neutraubling (DE)
(72) Erfinder: Braun, Franz, 93073 Neutraubling (DE); Söllner, Jürgen, 93073 Neutraubling (DE); Stammel, Volker, 93073 Neutraubling (DE); Meier, Robert, 93073 Neutraubling (DE)
(74) Vertreter: Bittner, Bernhard

(56) Entgegenhaltungen:
- EP-A1- 0 303 135
- WO-A2-2006/097243
- WO-A2-2009/024218
- WO-A2-2010/081866

## Beschreibung

Die vorliegende Erfindung bezieht sich auf ein Verfahren zum Sterilisieren von Behältnissen. In aseptischen Abfüllanlagen müssen die später zu befüllenden Behältnisse behandelt bzw. sterilisiert werden. Dies kann dabei bspw. durch fließfähige insbesondere gasförmige Medien geschehen. Da üblicherweise der Behandlungsprozess auf einer Kreisbahn bzw. während der Bewegung der Behältnisse auf einer Kreisbahn erfolgt und sich die Behältnisse bei der Behandlung vorteilhaft in Bewegung befinden, müssen die gasförmigen Medien von einer stehenden Leitung in mitrotierende Leitungen überführt werden. Dies kann bspw. mit einer Verteileinrichtung erfolgen, wie sie in der DE 10 2007 041 685 A1 beschrieben wurde. Der Gegenstand dieser Druckschrift wird hiermit vollständig zum Gegenstand auf der vorliegenden Anmeldung gemacht.

Oftmals werden für den Behandlungs- bzw. Sterilisationsprozess mehrere verschiedene insbesondere gasförmige Medien eingesetzt. Die Anzahl der Verteiler in der Anlage variiert dabei. Ein derartiger Behandlungsprozess ist in der Regel ein kontinuierlicher Prozess, d. h. Luft strömt dauerhaft durch den Verteiler. Diese Strömung wird in der Regel auch dann nicht unterbrochen, wenn sich keine Behältnisse unter den Behandlungsdüsen befinden. Dies ist vorteilhaft, um die Gesamtluftbilanz innerhalb eines Reinraums konstant zu halten. Eine Störung dieser Bilanz führt zu schwankenden Isolatordrücken die dann auch negative Werte, d. h. einen Unterdruck annehmen können.

Die WO 2006/097243 beschreibt ein Verfahren und eine Vorrichtung betreffend das Sterilabfüllen von Behältnissen. Aus der WO 2009/024218 A2 ist ein Dosier- und Versorgungssystem für Vorrichtungen zum H₂O₂-Sterilisieren von Packmitteln sowie eine Vorrichtung mit einem solchen Dosier- und Versorgungssystem bekannt.

Die EP 0 303 135 beschreibt ein Verfahren zum aseptischen bzw. sterilen Abfüllen von flüssigem Füllgut in Behälter sowie eine Vorrichtung zum Durchführen dieses Verfahrens.

Tritt also während der Produktion eine Störung auf, welche die Anlage in Stillstand versetzt, muss wegen der Gesamtluftbilanz weiter Luft in den Behandlungsraum einströmen. Da sich aber nun Behälter unter den Düsen befinden, würden diese Behälter überbehandelt werden, was unterbunden werden sollte. Eine derartige Verbindung sollte insbesondere dann erfolgen, wenn so überbehandelte Behältnisse nicht ausgeschleust werden können.

Aus der DE 10 2006 053 193 A1 ist eine Vorrichtung und ein Verfahren zur Herstellung von Kunststoffbehältnissen bekannt. Dabei ist vorgesehen, dass je nach herzustellender Flaschenart alternative Behandlungsmethoden angewandt werden. Werden bspw. Behältnisse für karbonisierte Getränke hergestellt, so wird eine Kühlbehandlung vorgesehen und werden Behältnisse für nicht karbonisierte Getränke hergestellt, so wird eine Sterilisationsbehandlung durchgeführt.

Ein anderes Anwendungsbeispiel sind kaltaseptische Abfüllanlagen. Dabei wird in der Regel ein steriles Produkt in eine vorher sterilisierte Flasche abgefüllt und mit einem sterilisierten Verschluss verschlossen. Die Sterilisation der Verpackungsmaterialien, die Abfüllung des Produkts sowie das Verschließen der Flaschen finden dabei meist in dieser Maschineneinheit statt. Der gesamte Raum, in dem die Sterilisation und auch die Abfüllung stattfindet wird dabei, sofern vorhanden, als Isolator bezeichnet.

Bei seitens der Anmelderin derzeit entwickelten Aseptikanlagen der neuesten Generation werden die Behältnisse mit einem gasförmigen Sterilisationsmedium sterilisiert. Dieses Sterilisationsmedium ist dabei üblicherweise ein Gemisch aus erwärmter Luft und einem verdampften Sterilisationsmittel, bei dem es sich bspw. um Wasserstoffperoxid oder Peressigsäure handeln kann.

Nachdem die Kunststoffbehältnisse mit diesem Sterilisationsmedium eine bestimmte Zeit sterilisiert wurden, können sie noch mehrere Male mit heißer Luft ausgeblasen werden, um die Rückstände an Sterilisationsmedium in diesen Behältnissen zu reduzieren.

Die Temperatur des Sterilisationsmediums und der Ausblasluft ist vorzugsweise relativ hoch (bspw. bei zwischen 70 ° und 130°). Auf diese Weise kann die Behandlungszeit reduziert werden. Weiterhin ermöglicht dies auch den Bau einer wirtschaftlichen Sterilisationsmaschine. Da allerdings die Behältnisse bei diesen Temperaturen schon zu schrumpfen beginnen, sollte vorzugsweise ein Optimum aus einerseits der Behandlungstemperatur und dem Flaschenschrumpf andererseits gefunden werden.

Diese Parameter erlauben in der Regel nur die durch die Maschinengeschwindigkeit definierte Behandlungszeit. Wird die Behandlungszeit überschritten, bspw. bei einem Maschinenstopp, werden die Behältnisse unter Umständen schon derart verformt, dass eine weitere Behandlung in der Maschine sogar zu mechanischen Schäden führen kann. Wie oben erwähnt, ist auch hier ein sofortiges Abschalten der Behandlungsmedien in der Regel nicht möglich, da sich dadurch die Behandlungsbedingungen in der Maschine derart verändern, dass ein Wiederanfahren mit einem sehr hohen Zeitaufwand und somit einem geringen Wirkungsgrad der Maschine verbunden wäre.

Der vorliegenden Erfindung liegt daher die Aufgabe zugrunde, ein Verfahren zur Behältnissterilisation zur Verfügung zu stellen, welches eine verbesserte Möglichkeit schafft, um bspw. auf Maschinenausfälle oder allgemein auf Diskontinuitäten im Maschinenlauf zu reagieren. Dies wird erfindungsgemäß durch ein Verfahren nach Anspruch 1 erreicht. Vorteilhafte Ausführungsformen und Weiterbildungen sind Gegenstand der Unteransprüche.

Die im Weiteren beschriebene Vorrichtung ist lediglich geeignet zur Durchführung des Verfahrens nach Anspruch 1.

Eine Vorrichtung zum Sterilisieren von Behältnissen weist eine Transporteinrichtung auf, welche die Behältnisse transportiert. Weiterhin Ist eine Sterilisationsmittelerzeugungseinrichtung vorgesehen, welche ein fließfähiges Sterilisationsmittel erzeugt sowie eine Zuführeinrichtung, welche das Sterilisationsmittel den Behältnissen zuführt.

Dabei weist die Vorrichtung eine Schalteinrichtung auf, um die Zuführung des Sterilisationsmittels zu den Behältnissen zu unterbrechen sowie eine Ableiteinrichtung, welche das Sterilisationsmittel derart ableitet, dass es nicht mehr unmittelbar den Behältnissen zugeführt wird.

Bevorzugt soll dabei eine Umschaltung derart möglich sein, dass die Sterilisationsmittelerzeugung selbst nicht abgeschaltet zu werden braucht und weiterhin das Sterilisationsmittel liefert. Die Schalteinrichtung und die Ableiteinrichtung können dabei im einfachsten Fall gemeinsam ausgeführt sein, bspw. in der Art eines Umlenkblechs, welches zwischen eine Ausslassöffnung der Zuführeinrichtung und das Behältnis eingeschoben wird.

Durch die erfindungsgemäße Vorgehensweise ist es möglich, die Sterilisationsmittelerzeugungseinrichtung weiterhin zu betreiben, andererseits jedoch das Sterilisationsmittel nicht mehr auf die Behältnisse gelangen zu lassen, so dass die Behältnisse nicht in irgendeiner Weise überbehandelt werden.

Bevorzugt ist die Transporteinrichtung derart ausgeführt, dass sie die Behältnisse vereinzelt transportiert. Dabei können beispielsweise Halteelemente vorgesehen sein, welche die einzelnen Behältnisse zum Zwecke ihres Transports halten, beispielsweise unterhalb eines Tragrings der Behältnisse oder an einem Boden der Behältnisse.

Vorteilhaft weist die Vorrichtung eine Vielzahl von Zuführeinrichtungen auf, welche die Behältnisse jeweils mit dem Sterilisationsmittel beaufschlagen. Dabei können diese Zuführeinrichtungen auch den jeweiligen Behältnissen bzw. Halteelementen für die Behältnisse zugeordnet sein. Diese Zuführeinrichtungen können dabei derart ausgeführt sein, dass sie das Sterilisationsmittel in die Behältnisse einführen und/oder derart, dass sie auch eine Aussenwandung der Behältnisse mit dem Sterilisationsmittel beaufschlagen.

Bei einer weiteren vorteilhaften Ausführungsform weist die Ableiteinrichtung eine Austrittsöffnung auf, welche weiter von den Behältnissen entfernt ist als eine Austrittsöffnung der Zuführleitung bzw. Zuführeinrichtung.

Bei dem Sterilisationsmittel handelt es sich insbesondere um ein Sterilisationsmittel, welches Wasserstoff oder Peressigsäure enthält.

Bei einer weiteren vorteilhaften Ausführungsform ist auch eine Vorrichtung zum Ausblasen der Behältnisse vorgesehen. Bei den Behältnissen handelt es sich insbesondere um Flaschen, bevorzugt Kunststoffflaschen, besonders bevorzugt PET-Flaschen, besonders bevorzugt PET-Flaschen mit einem Tragring, denkbar wären jedoch auch Verbundkartonverpackungen oder auch Glasflaschen. Es wäre jedoch auch möglich, dass es sich bei den Behältnissen um Kunststoffvorformlinge handelt, welche insbesondere durch einen Blasprozess in Kunststoffbehältnisse umgewandelt werden.

Bei einer vorteilhaften Ausführungsform handelt es sich bei der Ableiteinrichtung insbesondere um eine mit der Schalteinrichtung in Strömungsverbindung stehende Ableiteinrichtung.

Bei einer vorteilhaften Ausführungsform weist die Vorrichtung einen Sterilraum auf, durch den die Behältnisse während ihrer Sterilisation transportiert werden. Damit werden die Behältnisse durch den Sterilraum transportiert und vorteilhaft auch während dieses Transports sterilisiert insbesondere durch Beaufschlagung mit dem Sterilisationsmittel.

Bei einer weiteren vorteilhaften Ausführungsform weist die Sterilisationsmittelerzeugungseinrichtung eine Erwärmungseinrichtung auf, welche wenigstens einen Bestandteil des Sterilisationsmittels erwärmt. Insbesondere handelt es sich dabei um heiße Luft, die anschließend mit dem Sterilisationsmittel angereichert wird. Vorteilhaft stellt die Sterilisationsmittelerzeugungseinrichtung das Sterilisationsmittel mit Temperaturen zur Verfügung, welche zwischen 70° und 130°C liegen.

Wie oben erwähnt, sollte bei einem Maschinenstopp der Strom des heißen Behandlungsgases in die Behältnisse (zumindest teilweise) unterbunden werden, ohne dass dabei die Gesamtbilanz der in den Isolator zu- und abgeführten Medien verändert wird. Dies kann durch die erfindungsgemäß vorgeschlagene Ableitung des Sterilisationsgases vom regulären Weg in oder an die Flaschen auf einen alternativen Weg in einen anderen Bereich des Isolators unterbunden werden.

Wie noch genauer beschrieben wird kann es sich dabei bspw. bei der Schalteinrichtung um ein Umschaltventil bei oder in einer Rohrleitung handeln, über welches die Behandlungsmedien zugeführt werden. Bei einem Maschinenstau oder einer Änderung der normalen Betriebsgeschwindigkeit wird dieses Ventil umgeschaltet, so dass das entsprechende Behandlungsgas nicht mehr in oder an die Behältnisse (z.B. über eine Verteilvorrichtung) geleitet wird, sondern bspw. über einen separaten Anschluss direkt in den Isolator, d. h. den Reinraum strömt. Durch dieses Umschalten gelangt das Sterilisationsmedium nach wie vor in den Sterilraum und so bleibt die Gesamtbilanz der zu- und abgeführten Medien zum Isolator gleich und daneben werden auch die Behältnisse nicht mehr direkt einem z. B. heißen Gas ausgesetzt. Auf diese Weise wird ein unzulässiger Schrumpf der Behältnisse unterbunden. Vorteilhaft mündet daher die Ableitleitung in den Sterilraum. Mit anderen Worten gelangt unabhängig von einer Schaltstellung der Schalteinrichtung das Sterilisationsmedium vorteilhaft stets in den Sterilraum bzw. Reinraum (auch teilweise als Isolator bezeichnet). Das Sterilisationsmittel wird jedoch in diesem Falle nicht mehr unmittelbar auf die Behältnisse und insbesondere nicht auf deren Innenwandungen gerichtet.

Bei einer weiteren vorteilhaften Ausführungsform können auch mehrere Zuführeinrichtungen vorgesehen sein, welche das Sterilisationsmittel den Behältnissen zuführen sowie auch eine Verteileinrichtung, welche das Sterilisationsmittel auf die mehreren Zuführeinrichtungen verteilt. Besonders bevorzugt handelt es sich bei dieser Verteileinrichtung um einen Drehverteiler, der das Sterilisationsmittel von einem stationär angeordneten Reservoir auf eine Vielzahl von drehbaren Zuführleitungen aufteilt.

Dabei ist es auch möglich, dass die Ventileinrichtung bzw. Schalteinrichtung in diese Verteileinrichtung integriert ist und auf diese Weise bevorzugt die Zuführung des Sterilisationsmittels zu allen Zuführeinrichtungen unterbrechen kann.

Wie oben erwähnt, kann es sich jedoch bei der Ableiteinrichtung auch um eine mechanisch einschwenkbare Abschirmung handeln. Bei einer weiteren vorteilhaften Ausführungsform ist auch die Ableiteinrichtung selbst abschaltbar.

Während oben eine Bypasslösung beschrieben wurde, welche ggf. einen Mehraufwand darstellt, kann durch die Integration der Schalteinrichtung bzw. Ventileinrichtung in die Verteileinrichtung ggf. auf eine derartige Bypassleitung verzichtet werden, bzw. die Bypassleitung lediglich als Öffnung ausgeführt sein.

Bei dieser Ausführungsform mit der Verteilvorrichtung wird damit ein insbesondere gasförmiges Medium und insbesondere Sterilisationsmedium von einem stehenden Teil der Vorrichtung in ein drehendes Element der Vorrichtung verteilt, wobei die Vorrichtung weiterhin auch eine Bypassleitung aufweisen kann. Falls als Schalteinrichtung ein Umschaltventil oder bspw. ein an sich aus dem Stand der Technik bekanntes Regelventil eingesetzt wird, kann dieses in die Verteileinrichtung integriert sein. Vorteilhafter Weise finden sich diese Ventile in einer besonders bevorzugt stehenden Zuluftleitung.

Diese Ausführungsformen werden unten unter Bezugnahme auf die Figuren genauer erläutert.

Erfindungsgemäß wird bei Auftreten von vorgegebenen Zuständen mittels einer Schalteinrichtung die Zuführung des Sterilisationsmittels zu den Behältnissen unter wenigstens teilweiser Aufrechterhaltung der Sterilisationsmittelerzeugung unterbrochen und das Sterilisationsmittel wird mittels einer Ableiteinrichtung derart abgeleitet, dass es nicht mehr unmittelbar den Behältnissen zugeführt wird.

Wie oben erwähnt, handelt es sich bei diesen vorgegebenen Umständen insbesondere um Fehlerzustände, bei denen bevorzugt die Erzeugung des Sterilisationsmittels aufrechterhalten wird. Bevorzugt wird bei Auftreten der genannten Umstände eine Transportgeschwindigkeit der Behältnisse geändert und insbesondere verringert bzw. angehalten. Vorteilhaft werden die Behältnisse daher wenigstens zeitweise transportiert und vorzugsweise auch zeitweise während ihrer Sterilisation transportiert.

Vorteilhaft werden die Behältnisse während der Sterilisation entlang eines vorgegebenen Transportpfades und insbesondere durch einen Sterilraum transportiert.

Vorteilhaft wird bei Auftreten der vorgegebenen Zustände das Sterilisationsmittel weiterhin in diesen Sterilraum geleitet.

Vorteilhaft werden die Behältnisse wenigstens abschnittsweise entlang einer kreisförmigen Bahn transportiert und besonders bevorzugt auch während dieses Transports sterilisiert.

Bei einem weiteren vorteilhaften Verfahren wird das Sterilisationsmittel mittels einer Verteileinrichtung zumindest teilweise mehreren Behältnissen zeitgleich zugeführt und die Schalteinrichtung unterbricht bevorzugt die Zuführung des Sterilisationsmittels zu allen Behältnissen.

Weitere Vorteile und Ausführungsformen ergeben sich aus den beigefügten Zeichnungen:

Darin zeigen:
- Fig. 1: eine schematische Darstellung zur Veranschaulichung der vorliegenden Erfindung;
- Fig. 2: eine Skizze zur Veranschaulichung der vorliegenden Erfindung;
- Fig. 3a,b: zwei weitere Darstellungen einer Vorrichtung;
- Fig. 4a,b: zwei Darstellungen einer weiteren Ausführungsform einer Vorrichtung;
- Fig. 5: eine weitere Darstellung einer Vorrichtung;
- Fig. 6a,b: zwei weitere Darstellungen einer Vorrichtung;
- Fig. 7: eine weitere Ausführungsform einer Vorrichtung; und
- Fig. 8: eine weitere Ausführungsform einer Vorrichtung.

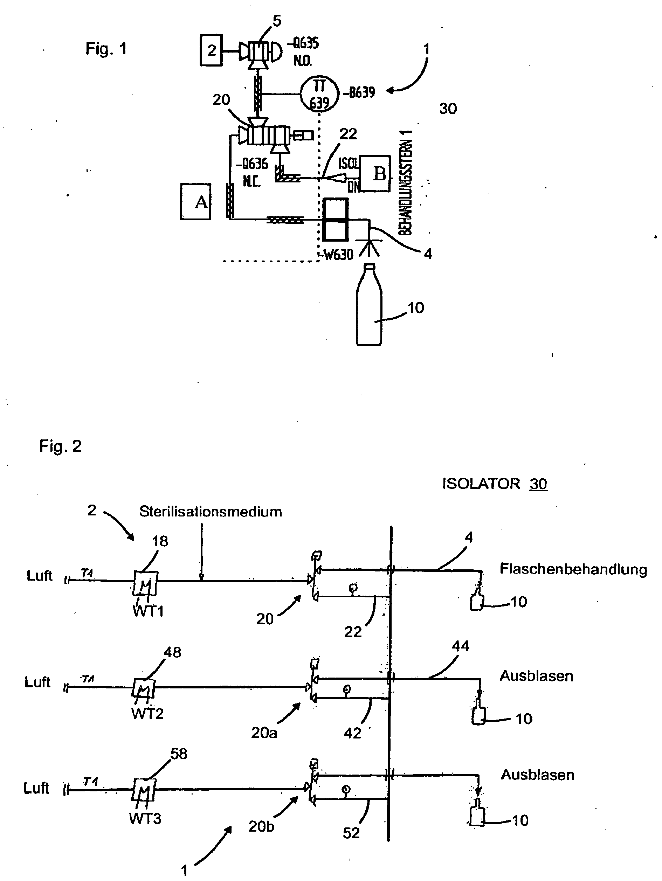

Fig. 1 zeigt eine schematische Darstellung einer Vorrichtung 1 zum Sterilisieren von Behältnissen 10. Dabei wird den zu sterilisierenden Behältnissen 10 ein Sterilisationsmedium über ein Regelventil 5 zugeführt und gelangt zunächst an eine Schalteinrichtung 20, die hier als Umschaltventil ausgeführt ist. Im normalen Arbeitsbetrieb ist diese Schalteinrichtung so eingestellt, dass das Sterilisationsmittel den Pfad A nimmt, d. h. über die Zuführleitung an bzw. in die Behältnisse 10 gelangt.

Bei einem weiteren Betriebszustand, bspw. einem Störzustand wird die Schalteinrichtung 20 umgeschaltet und das Sterilisationsmittel gelangt über einen Ableitkanal 22 bzw. einen Pfad B ebenfalls in den Reinraum 30, dabei jedoch nicht unmittelbar an und insbesondere in die Behältnisse 10. Das Bezugszeichen 2 kennzeichnet grob schematisch eine Sterilisationsmittelerzeugungseinrichtung.

Fig. 2 zeigt eine Skizze für eine Vorrichtung. Es wäre dabei auch möglich, dass die Schalteinrichtung 20 bzw. das Umschaltventil durch drei Absperrventile ersetzt wird. Um zu gewährleisten, dass die hydraulischen Bedingungen in der Zuführleitung 4 konstant bleiben, damit die Regler (für Luftmenge und Temperatur) in ihrem Arbeitsbereich konstant weiter arbeiten, ist es möglich, dass die Bypassleitung bzw. Ableitleitung 22 den gleichen Druckverlust aufweist wie der reguläre Weg zur Behandlung der Behältnisse. Zu diesem Zweck könnten Blenden in den jeweiligen Ableitsträngen vorgesehen sein. Vorteilhaft ist wenigstens ein derartiger Behandlungsstrang vorgesehen, es ist jedoch, insbesondere im Falle eines Drehverteilers eine Vielzahl derartiger Behandlungsstränge vorhanden.

Anstelle der in den Figuren 1 und 2 gezeigten Ausführungsformen könnte auch eine mechanische Abschirmung der Behältnisse von dem Behandlungsstrom (z. B. in Form eines einschwenkbaren Bleches) vorgesehen sein.

Das Bezugszeichen 18 kennzeichnet einen Wärmetauscher, der Luft erwärmt, damit der so erwärmten Luft anschließend ein Sterilisationsmedium zugeführt werden kann und das so entstehende Sterilisationsmedium bzw. Sterilisationsgemisch den Behältnissen zugeführt werden kann.

In einem weiteren, bzw. parallelen Strang wird ebenfalls Luft mittels eines weiteren Wärmetauschers erwärmt und die so erwärmte Luft den Behältnissen zum Ausblasen der Behältnisse (z.B. zum Zwecke des Trocknens) über eine Zuführleitung 44 zugeführt. Dabei ist hier eine weitere Schalteinrichtung 20a vorgesehen, die ebenfalls umgeschaltet werden kann, um die durch den Wärmetauscher 48 erwärmte Luft von den Behältnissen wegzuleiten. Bevorzugt wird jedoch auch diese so über eine Ableitung 42 abgeleitete Luft in den Sterilraum 30 geleitet. Es wäre jedoch auch möglich, dass die Ableitung 42 nicht in dem Sterilraum endet.

Das Bezugszeichen 58 bezieht sich auf einen weiteren Wärmetauscher, der ebenfalls zum Erwärmen von Luft dient. Auch hier ist wieder eine Schalteinrichtung 20b vorgesehen, welche dazu dient, um die erwärmte Luft im Bedarfsfall von den Behältnissen über eine Ableitung 52 wegzuleiten. Durch die hier beschriebenen Schalteinrichtungen 20a und 20b kann - insbesondere bei Auftreten von Fehlern - gleichwohl der Wärmetauscher 48 bzw. 58 weiter betrieben werden.

Durch die Erfindung ist es möglich, eine Maschine anzuhalten, ohne dass dabei sich in der Maschine befindliche Flaschen verformt werden. Daneben ist auch ein sofortiges Wiederanfahren nach Behebung eines Fehlerzustandes, der zum Stillstand führte, möglich. Ohne die Schaltvorrichtung 20 müsste die Medienversorgung bspw. ein Sterilisationsgaserzeuger abgeschaltet werden, was in der erforderlichen Zeit kaum möglich ist. Danach könnte nicht weiter produziert werden sondern man müsste warten, bis sich die entsprechenden Parameter wieder eingestellt haben. Weil dann aber zuerst die Maschine leergefahren werden muss, gelangen auch unsterile Behältnisse in den Isolator, wodurch dieser unsteril wird und vor einer Fortsetzung der Produktion erst wieder resterilisiert werden muss. Durch die erfindungsgemäße Vorgehensweise kann ein kontinuierlicher Betrieb weitgehend nach Behebung eines Fehlers aufrechterhalten werden.

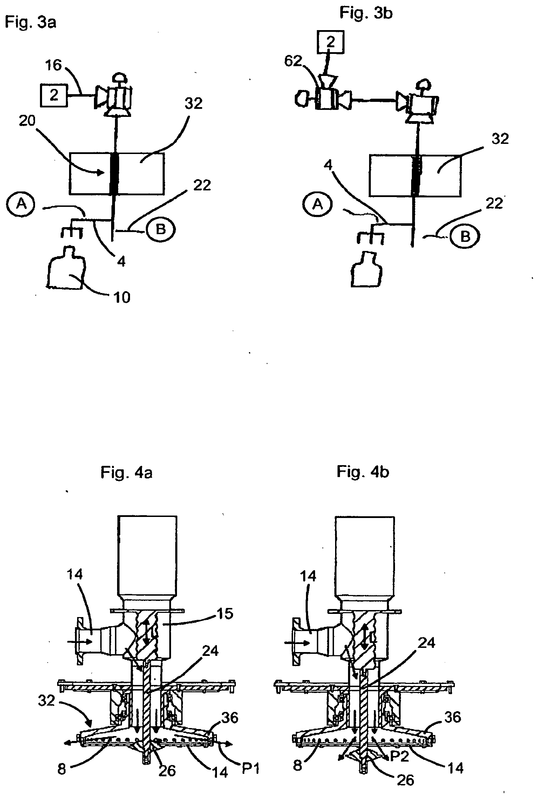

Die Figuren 3a und 3b veranschaulichen grob schematisch eine weitere Ausführungsform. In diesem Fall ist eine Verteileinrichtung 32 vorgesehen bzw. ein Verfahren, welches gasförmige Medien von einem stehenden in ein drehendes Teil verteilt und in der vorteilhaft gleichzeitig eine Bypassleitung als eine Ableitleitung zumindest abschnittsweise integriert ist. Dabei wäre es möglich, sowohl die Schalteinrichtung 20 als auch ein Regelventil 5 (vergleiche Fig. 1) in die Verteileinrichtung zu integrieren. Vorteilhaft befinden sich damit das oder die benötigten Ventile in einer stehenden Zuluftleitung 16.

Dabei ist es möglich, dass eine Schalteinrichtung als Ventil ausgeführt wird, welches bspw. einen bewegbaren Kolben aufweist, wobei so durch den Hub einer Ventilstange ein Luftweg freigegeben wird, so dass das gasförmige Medium nicht mehr den Behältnissen sondern nur noch dem Isolator zugeführt wird (Pfad B). Vorteilhaft ist dabei der Widerstand des freigegebenen Luftwegs geringer als der Luftweg zu den jeweiligen Behandlungsdüsen. Die Behältnisse werden dann nicht mehr behandelt und die Luftbilanz des Reinraums 30 bzw. Isolators bleibt gleichwohl konstant. Auf eine Bypassleitung wie bspw. in Fig. 1 veranschaulicht, kann in diesem Falle verzichtet werden.

Weiterhin wäre es jedoch auch möglich, mit dem Hub der Ventilstange zusätzlich den durchströmenden Volumenstrom zu regeln. Dabei könnte ein zusätzlicher Ventilsitz im stehenden Teil der Verteileinrichtung 32 vorgesehen sein. In Fig. 1 bis 3 kennzeichnet das Bezugszeichen A jeweils den Luft- bzw. Sterilisationsmittelweg im Arbeitsbetrieb bzw. für die Behandlung der Behältnisse und das Bezugszeichen B den Luftweg bei einem Bypass. Bei der in Fig. 3b gezeigten Darstellung ist ein zusätzliches Regelventil 62 vorgesehen.

Die Figuren 4a und 4b zeigen eine weitere mögliche Ausführungsform. Dabei werden die Gase bzw. Sterilisationsmedien hier seitlich über eine Zuführleitung 14 zugeführt und innerhalb eines Ventilraums 15 nach unten umgelenkt. Weiterhin ist eine Ventilstange 24 vorgesehen, an der ein Ventilkörper 26 bzw. Ventilkegel angeordnet ist. Damit ist auch dieser Ventilkörper (insbesondere in der Längsrichtung der Ventilstange 24) beweglich. Dabei ist diese Ventilstange 24 so ausgeprägt, dass der Ventilkegel 26 einstell- und auswechselbar ist. Bei der Montage kann die Position des Ventilkegels 26 in der Längsrichtung L eingestellt werden. Im Betrieb kann der Ventilkegel 26 auch in dieser Richtung bewegt werden.

Eine Abdichtung zwischen drehenden und stehenden Teilen kann hier durch Überströmspalte erfolgen. Es werden jedoch auch andere Mittel für die Abdichtung möglich wie bspw. hydraulische Dichtungen oder Gleitringdichtungen.

Durch den austauschbaren Ventilkörper bzw. Ventilkegel 26 kann an dieser Stelle auch eine berührende Dichtung verwendet werden. Das Bezugszeichen 8 bezieht sich auf Öffnungen, durch welche das Sterilisationsmedium aus der Verteileinrichtung 32 austreten kann. An diesen Öffnungen können sich Zuführleitungen (nicht gezeigt) befinden, welche das Sterilisationsmedium den einzelnen Behältnissen zuführen. Das Bezugszeichen 36 kennzeichnet einen Deckel der Verteileinrichtung 32, der sich mit dreht. Auch ein Bodenteil 14 kann sich bei der in Figuren 4a und 4b gezeigten Ausführungsform mit drehen (um die Längsrichtung L).

Das Bezugszeichen P1 kennzeichnet die Ausströmrichtung des Sterilisationsmediums, welches auf diese Weise zu den Behältnissen gelangt. In Fig. 4b kennzeichnet das Bezugszeichen P2 die Ausströmrichtung wenn der Ventilkegel geöffnet ist, d. h. bspw. in einem Fehlerzustand. In diesem Fall gelangt das Sterilisationsmedium nicht zu den einzelnen Behältnissen sondern unmittelbar in den Reinraum 30. Bei dem Ventil selbst kann es sich um ein hydraulisches, pneumatisches oder auch elektrisch betätigtes Ventil handeln.

Damit ist bei der in Fig. 4a gezeigten Situation während der Behältnisbehandlung die Ventilstange 24 eingefahren und damit die Bohrung innerhalb des Ventildeckels bzw. des Bodenteils 14 verdeckt. Das Sterilisationsmittel strömt damit durch die einzelnen Öffnungen und (nicht gezeigte) Leitungen in (ebenfalls nicht gezeigte) Behandlungsdüsen und gelangt danach in die Behältnisse. Das durch die Spaltdichtungen der Vorrichtung strömende Gas kann ebenfalls in den Behandlungs- bzw. Sterilraum 30 gelangen und dient dort zusammen mit dem aus den Behältnissen strömenden Gas zur Außenbehandlung der Behältnisse. Das Bezugszeichen 36 kennzeichnet ein (insbesondere drehbares) Deckelteil der Verteileinrichtung 32.

Bei der in Fig. 4b gezeigten Situation ist das Ventil geöffnet und das Sterilisationsmedium kann entsprechend durch den Bypass (Pfeil P2) austreten.

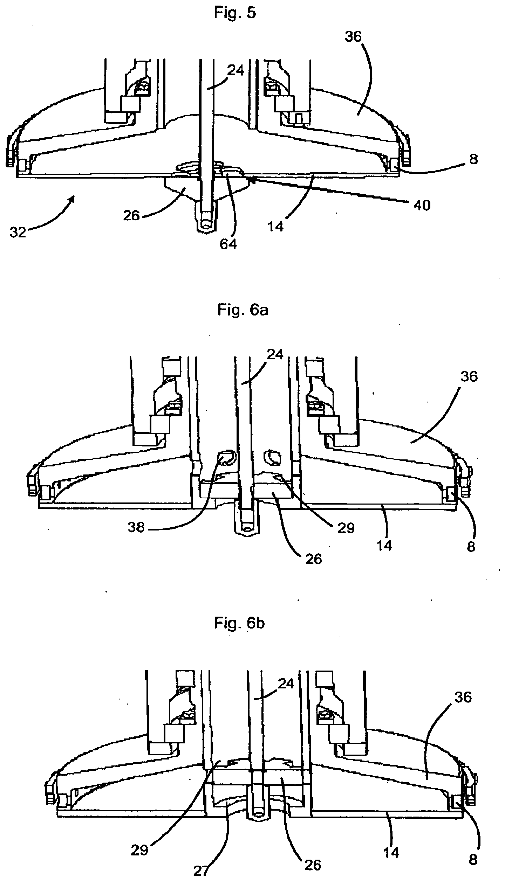

Bei der in Fig. 5 gezeigten Ausführungsform ist die Ventilstange 24 zusätzlich im drehenden Teil, d. h. hier des Bodenteils 14 geführt. Die Abdichtung des Bypassweges erfolgt hier ebenfalls axial. Entsprechend bezieht sich das Bezugszeichen 40 in seiner Gesamtheit auf eine axiale Abdichtung. Bei geöffnetem Ventil kann das Sterilisationsmittel unmittelbar durch die Öffnungen 64 aus der Verteileinrichtung 32 in den Reinraum 30 austreten.

Die Figuren 6a und 6b zeigen eine weitere Ausführungsform der Vorrichtung. Auch hier ist eine Luftwegeregelung im stehenden Teil angeordnet. Bei der in Fig. 6a gezeigten Ausführungsform ist der Luftweg für die Behältnisbehandlung freigegeben. Das Sterilisationsmittel kann durch die Öffnungen 38 zu den Öffnungen 8 gelangen und über diese austreten. Nach unten kann ein Gas hier nicht austreten, da die Öffnungen 29, die in dem Ventilkörper angeordnet sind, verdeckt sind da der Ventilkörper 26 auf einen ringförmigen Abschnitt 27 aufliegt.

Bei der in Fig. 6b gezeigten Situation ist die Ventilstange 24 (und damit auch der Ventilkörper) nach oben geschoben. Durch den hier sternförmigen Ventilkörper wird der Luftweg für die Behältnisbehandlung verdeckt. Genauer werden die Öffnungen 38 verdeckt. Durch die regelmäßig am Umfang angeordneten Ausschnitte 29 kann das Sterilisationsmedium nun über den Bypassweg in den Sterilisationsraum strömen. Bei der hier gezeigten Ausführungsform sowie bei den oben gezeigten Ausführungsformen ist daher vorteilhaft die Ventileinrichtung radial innerhalb von Austrittsöffnungen für das Sterilisationsmedium vorgesehen. Vorteilhaft weist die Schalteinrichtung bzw. die Ventileinrichtung einen Ventilkörper auf, der in Richtung der Drehachse der Verteileinrichtung bewegbar ist.

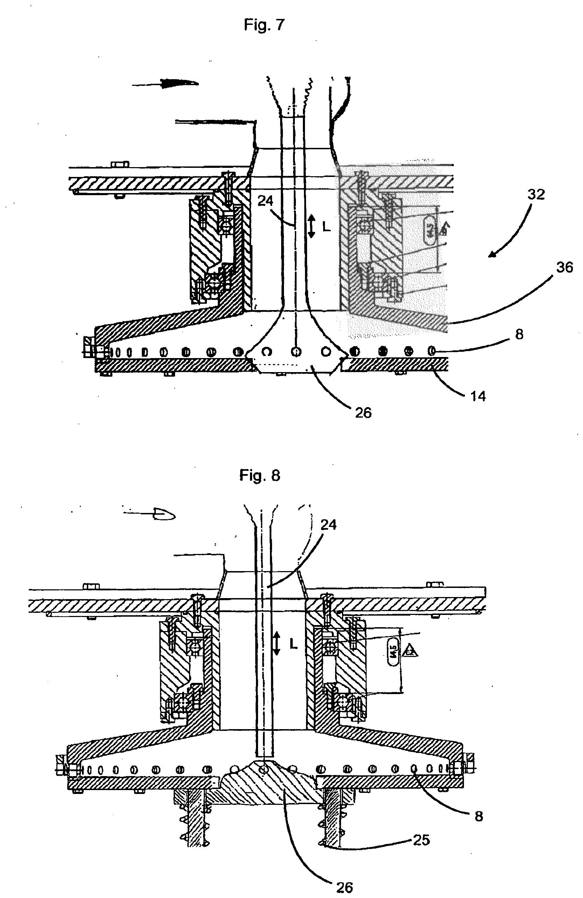

Bei der in Fig. 7 gezeigten Ausführungsform ist der Ventilkegel bzw. Ventilkörper innerhalb der Verteileinrichtung 32 angeordnet. Bei dieser Ausführungsform erfolgt ein Schließen des Ventils durch ein Herabsenken des Ventilkörpers 26.

Fig. 8 zeigt eine weitere Ausführungsform einer Vorrichtung. Bei dieser Ausführungsform ist der Ventilkörper bzw. Ventilkegel wieder unterhalb der Platte bzw. des Bodenteils 14 angeordnet so dass ein Öffnen des Ventils hier wieder dadurch stattfindet, dass der Ventilkörper nach unten gedrückt wird. Weiterhin ist eine Feder 25 vorgesehen, welche den Ventilkörper wiederum nach oben vorspannt. Die Ventilstange 24 befindet sich hier im stehenden Teil der Verteileinrichtung 32 und der Ventilkörper 26 im drehenden Teil. Während des normalen Arbeitsbetriebs bzw. während der Behandlung ist zwischen der Ventilstange 24 und dem Ventilkörper ein Luftspalt ausgebildet. Vorzugsweise ist daher bei dieser Ausführungsform der Ventilkörper 26 von der Ventilstange wenigstens zeitweise und insbesondere in einem geschlossenen Zustand des Ventilkörpers 26 entkoppelt.

Der Ventilkörper wird damit durch die Feder 25 an dem ebenfalls mitdrehenden Deckel 14 angedrückt. Zum Öffnen des Bypassweges drückt die Ventilstange den Ventilkörper nach unten und gibt damit den Weg frei.

Bei einer weiteren (nicht gezeigten) Ausführungsform kann die Schalt- bzw. Ventileinrichtung vor der Verteileinrichtung auch an einem Teilstück der Zuführleitung innerhalb des Isolators angeordnet sein und dabei einfach als verschiebbare Hülse auf oder innerhalb der Zuführleitung ausgeprägt sein. Dabei sind in der Wandung der Zuführleitung Austrittsöffnungen angeordnet, die im normalen Betrieb der Sterilisationsvorrichtung durch die verschiebbare Hülse verdeckt und damit verschlossen sind. Bei einem Störungszustand der Sterilisationsvorrichtung wird die Hülse durch einen geeigneten Antrieb derart verschoben, dass die Öffnungen in der Wandung freigegeben werden und als Ableiteinrichtung für das gasförmige Medium in den Isolator wirken.

Bei einer weiteren (nicht gezeigten) Ausführungsform könnte sich das Ventil auch im drehenden Teil oder im Behandlungsraum selbst befinden. Es wäre jedoch auch möglich, statt eines Ventils andere Antriebe vorzusehen wie bspw. elektromagnetische Spindelmotoren und dergleichen. Daneben wäre es auch denkbar, dass das Ventil gleichzeitig als Regelventil ausgebildet wird, wobei durch entsprechende Zwischenstellungen des Ventilkörpers 26 auch festgelegt werden kann, dass bestimmte Anteile des Sterilisationsmediums durch die Öffnungen 8 gelangen, während andere direkt nach unten austreten.

### Bezugszeichenliste

- 1: Vorrichtung
- 2: Sterilisationsmittelerzeugungseinrichtung
- 4: Zuführeinrichtung
- 8: Öffnungen
- 5: Regelventil
- 10: Behältnis
- 14: Bodenteil
- 15: Ventilraum
- 16: Zuluftleitung
- 18: Wärmetauscher
- 20: Schalteinrichtung
- 20a: Schalteinrichtung
- 20b: Schalteinrichtung
- 22: Ableiteinrichtung, Ableitleitung
- 24: Ventilstange
- 25: Feder
- 26: Ventilkörper, Ventilkegel
- 27: ringförmiger Abschnitt
- 29: Ausschnitt
- 30: Reinraum
- 32: Verteileinrichtung
- 36: Deckelteil
- 38: Öffnungen
- 40: axiale Abdichtung
- 42: Ableitung
- 44: weitere Zuführleitung (für Heißluft)
- 48: Wärmetauscher
- 52: Ableitung
- 58: Wärmetauscher
- 62: Regelventil
- 64: Öffnungen
- A, B: Pfade für das Sterilisationsmittel
- P1, P2: Strömungsrichtungen

## Patentansprüche

1. Verfahren zum Sterilisieren von Behältnissen (10), wobei mittels einer Sterilisationsmittelerzeugungseinrichtung (2) ein fließfähiges Sterilisationsmittel erzeugt wird und dieses Sterilisationsmittel mittels einer Zuführeinrichtung (4) den Behältnissen (10) in einem Sterilraum (30), durch den die Behältnisse (10) während ihrer Sterilisation transportiert werden, zugeführt wird,
**dadurch gekennzeichnet, dass**
bei Auftreten von vorgegebenen Zuständen mittels einer Schalteinrichtung (20) die Zuführung des Sterilisationsmittels zu den Behältnissen (10) unter wenigstens teilweiser Aufrechterhaltung der Sterilisationsmittelerzeugung unterbrochen wird und das Sterilisationsmittel mittels einer Ableiteinrichtung derart abgeleitet wird, dass es nicht mehr unmittelbar den Behältnissen (10) zugeführt wird.

2. Verfahren nach Anspruch 1,
**dadurch gekennzeichnet, dass**
die Behältnisse (10) während der Sterilisation entlang eines vorgegebenen Transportpfades, insbesondere durch einen Sterilraum (30) transportiert werden.

3. Verfahren nach wenigstens einem der vorangegangenen Ansprüche,
**dadurch gekennzeichnet, dass**
das Sterilisationsmittel mittels einer Verteileinrichtung (32) zumindest zeitweise mehreren Behältnissen zeitgleich zugeführt wird und die Schalteinrichtung (20) die Zuführung des Sterilisationsmittels zu allen Behältnissen (10) unterbricht.

## Claims

1. A method of sterilizing containers (10), wherein a flowable sterilization agent is produced by means of a sterilization agent production device (2) and this sterilization agent is supplied by means of a supply device (4) to the containers (10) in a sterile room (30) through which the containers (10) are conveyed during the sterilization thereof,
**characterized in that**
if pre-determined states occur the supply of the sterilization agent to the containers (10) is interrupted by means of a switching device (20) whilst maintaining the production of the sterilization agent at least in part and the sterilization agent is drawn away by means of a drawing-away device in such a way that it is no longer supplied directly to the containers (10).

2. A method according to claim 1,
**characterized in that**
during the sterilization the containers (10) are conveyed along a pre-determined conveying path, in particular through a sterile room (30).

3. A method according to at least one of the preceding claims,
**characterized in that**
the sterilization agent is supplied simultaneously to a plurality of containers at least for a time by means of a distribution device (32), and the switching device (20) interrupts the supply of the sterilization agent to all the containers (10).

## Revendications

1. Procédé de stérilisation de récipients (10), où un agent de stérilisation fluide est produit au moyen d'un dispositif (2) de génération d'agent de stérilisation, et où ledit agent de stérilisation est conduit par un dispositif d'amenée (4) vers les récipients (10) dans un espace stérile (30) que traversent les récipients (10) transportés pendant leur stérilisation,
**caractérisé en ce qu'**
à l'apparition de circonstances définies, la conduction de l'agent de stérilisation vers les récipients (10) est interrompue au moyen d'un dispositif de commutation (20), la génération de l'agent de stérilisation étant au moins partiellement maintenue, et **en ce que** l'agent de stérilisation est dérivé par un dispositif de dérivation (1) de manière à ne plus être directement conduit vers les récipients (10).

2. Procédé selon la revendication 1,
**caractérisé en ce que**
pendant la stérilisation, les récipients (10) sont transportés le long d'un chemin de transport défini, traversant en particulier un espace stérile (30).

3. Procédé selon au moins une des revendications précédentes,
**caractérisé en ce que**
l'agent de stérilisation est simultanément conduit au moins temporairement vers plusieurs récipients au moyen d'un dispositif distributeur (32), et **en ce que** le dispositif de commutation (20) interrompt la conduction de l'agent de stérilisation vers tous les récipients (10).
